# EUROPEAN PATENT SPECIFICATION

(11) **EP 3 135 200 B1**
(45) Date of publication and mention of the grant of the patent: **02.10.2019**
(21) Application number: 15782885.6
(22) Date of filing: 04.03.2015
(51) Int. Cl.: A61B 6/00

(54) **DUAL-PLANE X-RAY IMAGING APPARATUS**
RÖNTGENBILDGEBUNGSVORRICHTUNG MIT DUALER EBENE
APPAREIL D'IMAGERIE À RAYONS X À DEUX PLANS

(30) Priority: 22.04.2014 CN 201410161383
(43) Date of publication of application: 01.03.2017
(73) Proprietor: Beijing East Whale Image Technology Co., Ltd., Haidian District, Beijing 100176 (CN)
(72) Inventor: TIAN, Chunlei, Beijing 100176 (CN); ZHANG, Jun, Beijing 100176 (CN); CAO, Liu, Beijing 100176 (CN)
(74) Representative: Hanna Moore + Curley
(86) International application number: PCT/CN2015/000131
(87) International publication number: WO 2015/161669

(56) References cited:
- WO-A2-2007/029202
- CN-A- 1 220 134
- CN-A- 103 784 155
- CN-A- 103 800 021
- CN-A- 103 908 275
- CN-U- 202 960 537
- CN-U- 203 220 375
- CN-U- 203 220 379
- US-A- 5 515 416
- US-A1- 2004 202 285
- US-A1- 2015 036 799

## Description

### Field of technology

This invention relates to an X-ray imaging device, especially a biplanar X-ray imaging device.

### Background

The biplanar imaging device has many advantages such as determining the position of patients more accurately, saving the time of operation for doctors, reducing the exposure dose and maintaining the sterile environment, so it is widely used in operations.

A biplanar imaging device can be realized through the device in US Patent US4884293 which describes a biplanar imaging system where one imaging system is fixed on the ground of the operating room and the other imaging system is hanged on the guide rail of the ceiling. The disadvantage of the device is that the two imaging systems should be matched accurately upon initial installation, which may easily lead to the result that the two imaging systems have different rotating axes.

The said biplanar imaging device can also be realized through the device in US Patent US3549885 which describes a biplanar imaging system where two imaging systems are installed in a rotatable circular ring orthogonally. The disadvantage of this device is that the angle between the two imaging systems is always 90 degrees and can't be changed.

The said biplanar imaging device can also be realized through the device in US Patent US5095501 where two imaging systems are installed in a rotatable 3/4 circular ring orthogonally to form a rotatable G-shaped arm. The disadvantage of this device is that the angle between the two imaging systems is always 90 degrees and can't be changed.

The document CN 103800021 A discloses an X-ray out-phase pulse biplanar synchronous real-time imaging device. The document US 5,515,416 discloses a system having two radiation axis with a common isocenter for simultaneous viewing of a targeted object in two planes. The document US 2004/0202285 A1, discloses a C-arm assembly for multiple imaging of a target. The document US 2015/0036799 A1 discloses an X-ray apparatus.

### Summary of the invention

This invention is designed to provide a biplanar X-ray imaging device which can form biplanar and multi-angle images of objects through the interactions between two imaging systems and their rotations and movements as a whole.

This invention adopts the following technical solutions to solve the technical problems: a biplanar X-ray imaging device, comprising the first C-shaped arm, the second C-shaped arm, a sliding and rotating seat, the first X-ray generation device, the first image receiving device, the second X-ray generation device and the second image receiving device; the first C-shaped arm is slidably disposed outside the second C-shaped arm; the second C-shaped arm is slidably disposed on the sliding and rotating seat; the first C-shaped arm (1) consists of two C-shaped beams ; a chute is disposed on the second C-shaped arm (2), which matches the C-shaped beam of the first C-shaped arm (1); the first X-ray generation device is fixed on one end in the peripheral direction of the first C-shaped arm; the first image receiving device is disposed on the other end in the peripheral direction of the first C-shaped arm or slidably disposed on the inner side of the second C-shaped arm; the second X-ray generation device and the second image receiving device are respectively fixed on the two ends in the peripheral direction of the second C-shaped arm. The intersecting line between the X-ray emission plane of the first X-ray generation device and the X-ray emission plane of the second X-ray generation device is the rotating axis; the rotating axis is on the connecting line of the first X-ray generation device and the first image receiving device. In addition, the second X-ray generation device and the second image receiving device rotate along the rotating axis under the drive of the second C-shaped arm.

Alternatively, the biplanar X-ray imaging device also comprises: a driving device disposed on the second C-shaped arm to drive the first C-shaped arm to slide along the second C-shaped arm;
Alternatively, there are two driving devices respectively disposed on the second C-shaped arm to drive the first C-shaped arm and the first image receiving device respectively to slide along the second C-shaped arm.

Alternatively, the biplanar X-ray imaging device also comprises lifting and rotating components, an operating arm and a mobile trolley; the sliding and rotating seat is disposed on the operating arm and can slide and rotate relative to the operating arm; the operating arm is disposed on the mobile trolley through the lifting and rotating components.

Alternatively, the biplanar X-ray imaging device also comprises lifting and rotating components, an operating arm and the first base; the sliding and rotating seat is disposed on the operating arm and can slide and rotate relative to the operating arm; the operating arm is disposed on the first base through the lifting and rotating components.

Alternatively, the biplanar X-ray imaging device also comprises lifting and rotating components, an operating arm and the second base; the sliding and rotating seat is disposed on the operating arm and can slide and rotate relative to the operating arm; the operating arm is disposed on the second base through the lifting and rotating components; the second base is slidably disposed on the ceiling.

This invention has the following advantageous effects: the biplanar X-ray imaging device of this invention comprises the second C-shaped arm and the first C-shaped arm; the first X-ray generation device and the first image receiving device are installed on the two ends of the first C-shaped arm, or the first X-ray generation device is installed on one end of the first C-shaped arm and the first image receiving device is disposed on the inner side of the second C-shaped arm; the first X-ray generation device and the first image receiving device constitute the first imaging device; the second X-ray generation device and the second image receiving device are installed on both ends of the second C-shaped arm; the second X-ray generation device and the second image receiving device constitute the second imaging device; when the first C-shaped arm slides along the second C-shaped arm, different angles are formed between the first imaging device and the second imaging device, so that images of the detected object can be formed from different angles; the first C-shaped arm and the second C-shaped arm share the same rotating axis, which ensures that the first imaging device and the second imaging device share the same rotating axis so that biplanar and motile-angle images of the object can be formed.

### Brief description of the drawings

Figure 1 shows the structure of a first biplanar X-ray imaging device not forming part of this invention;
Figure 2 shows the structure of a second biplanar X-ray imaging device not forming part of this invention;
Figure 3 shows the structure of a third biplanar X-ray imaging device not forming part of this invention;
Figure 4 shows the structures of the first C-shaped arm and the second C-shaped arm of a biplanar X-ray imaging device not forming part of this invention;
Figure 5 shows the structures of the first C-shaped arm and the second C-shaped arm of the biplanar X-ray imaging device similar to the structure of this invention;
Figure 6 shows the structure of the first C-shaped arm and the section of the second C-shaped arm of biplanar X-ray imaging device not forming part of this invention;
Figure 7 shows the structure of the first C-shaped arm and the section of the second C-shaped arm of a biplanar X-ray imaging device not forming part of this invention;
Figure 8 shows the structure of the C-shaped arm and the section of the second C-shaped arm of a biplanar X-ray imaging device not forming part of this invention;
Figure 9 shows the structure of the first C-shaped arm and the section of the second C-shaped arm of a biplanar X-ray imaging device not forming part of this invention.

Labels in figures: 1 - the first C-shaped arm; 2 - the second C-shaped arm; 3 - the sliding and rotating seat; 4 - the first X-ray generation device; 5 - the first image receiving device; 6 - the second X-ray generation device; 7 - the second image receiving device; 8 - supporting device; 9 - driving device; 10 - lifting and rotating components; 11 - operating arm; 12 - mobile trolley; 13 - the first base; 14 - the second base.

### Detailed description of preferred embodiments

The following part further clarifies the technical solutions of this invention in combination with embodiments and attached figures.

### Embodiment 1

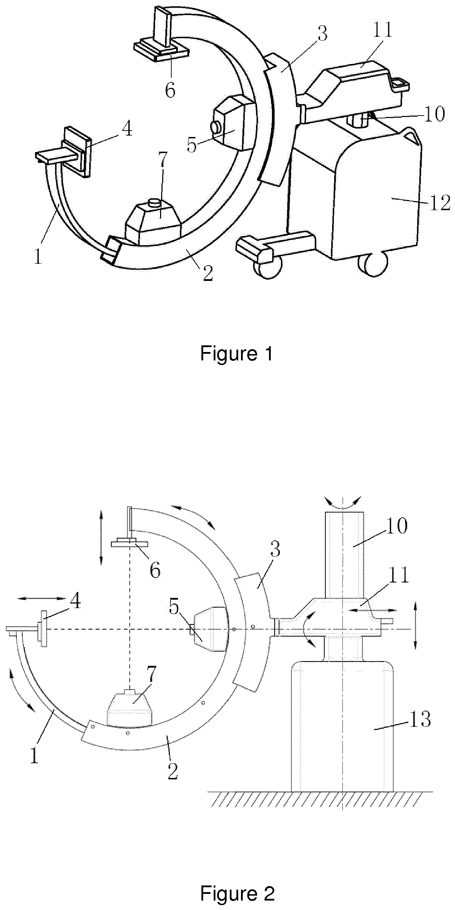

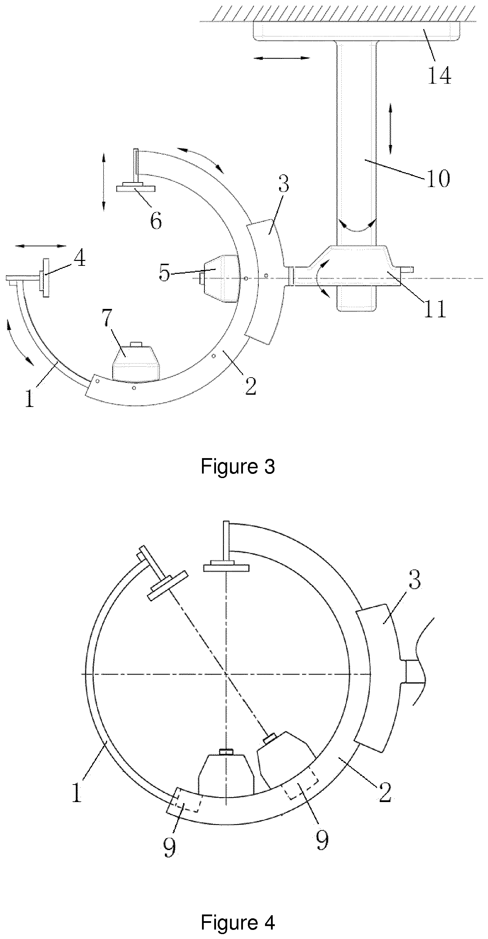

See Figures 1-9. This embodiment which is not forming part of the present invention provides a biplanar X-ray imaging device, comprising the first C-shaped arm 1, the second C-shaped arm 2, the sliding and rotating seat 3, the first X-ray generation device 4, the first image receiving device 5, the second X-ray generation device 6 and the second image receiving device 7;
The first C-shaped arm 1 is slidably disposed on the second C-shaped arm 2 through the supporting device 8; the second C-shaped arm 2 is slidably disposed on the sliding and rotating seat 3;
The first X-ray generation device 4 is fixed on one end in the peripheral direction of the first C-shaped arm 1;
The first image receiving device 5 is disposed on the other end in the peripheral direction of the first C-shaped arm 1 or slidably disposed on the inner side of the second C-shaped arm 2;
The second X-ray generation device 6 and the second image receiving device 7 are respectively fixed on the two ends in the peripheral direction of the second C-shaped arm 2. The intersecting line between the X-ray emission plane of the first X-ray generation device 4 and the X-ray emission plane of the second X-ray generation device 6 is the rotating axis;
The rotating axis is on the connecting line of the first X-ray generation device 4 and the first image receiving device 5. In addition, the second X-ray generation device 6 and the second image receiving device 7 rotate along the rotating axis under the drive of the second C-shaped arm 2.

The biplanar X-ray imaging device of this embodiment which is not forming part of the present invention comprises: the second C-shaped arm 2 and the first C-shaped arm 1 which can be slidably disposed within the second C-shaped arm 2; the first X-ray generation device 4 and the first image receiving device 5 are installed on the two ends of the first C-shaped arm 1, or the first X-ray generation device 4 is installed on one end of the first C-shaped arm 1 and the first image receiving device 5 is disposed on the inner side of the second C-shaped arm 2; the first X-ray generation device 4 and the first image receiving device 5 constitute the first imaging device; the second X-ray generation device 6 and the second image receiving device 7 are installed on both ends of the second C-shaped arm 2; the second X-ray generation device 6 and the second image receiving device 7 constitute the second imaging device; when the first C-shaped arm 1 slides along the second C-shaped arm 2, different angles are formed between the first imaging device and the second imaging device, so that images of the detected object can be formed from different angles; the first C-shaped arm 1 and the second C-shaped arm 2 share the same rotating axis, which ensures that the first imaging device and the second imaging device share the same rotating axis so that biplanar and motile-angle images of the object can be formed.

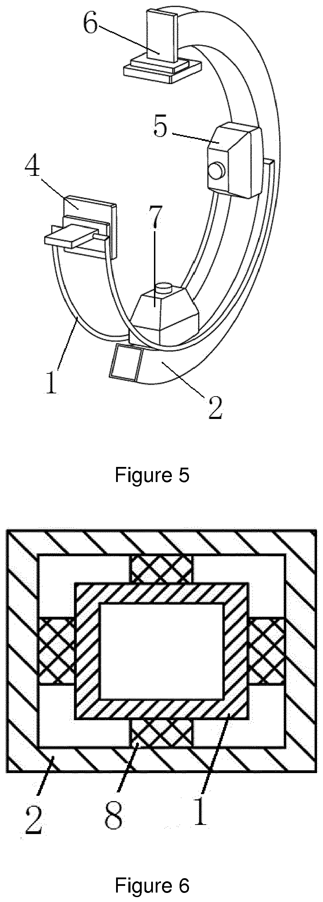

See Figure 6. In this embodiment, which is not forming part of the present invention, alternatively, both the sections of the first C-shaped arm 1 and the second C-shaped arm 2 are rectangles, and there are four supporting devices 8 between the first C-shaped arm 1 and the second C-shaped arm 2. The four supporting devices 8 are respectively disposed on the outer surface of the first C-shaped arm 1, and the four supporting devices 8 are respectively disposed on the four sides of the rectangle of the first C-shaped arm 1, so that the second C-shaped arm 2 can support the first C-shaped arm 1 through the four supporting devices 8; besides, with the selection of materials of the supporting devices 8, such as ceramic, the supporting devices 8 can produce the technical effect of friction reduction.

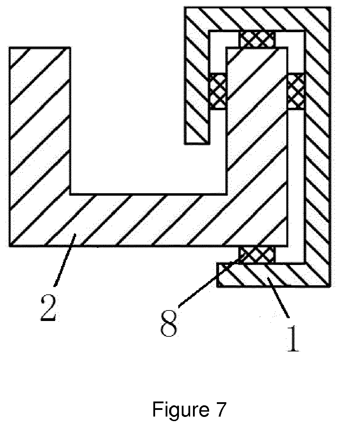

See Figure 7. In this embodiment, which is not forming part of the present invention, alternatively, the section of the second C-shaped arm is " 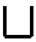 "; the section of the first C-shaped arm is " 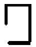 "; there are four supporting devices between the first C-shaped arm and the second C-shaped arm; the four supporting devices are disposed in the position between the part of the second C-shaped arm which is within the first C-shaped arm and the first C-shaped arm, i.e. the four sides (upside, downside, left side and right side) of the part of the second C-shaped arm which is within the first C-shaped arm; the second C-shaped arm support the first C-shaped arm through the four supporting devices; besides, with the selection of materials of the supporting devices, such as ceramic, the supporting devices can produce the technical effect of friction reduction.

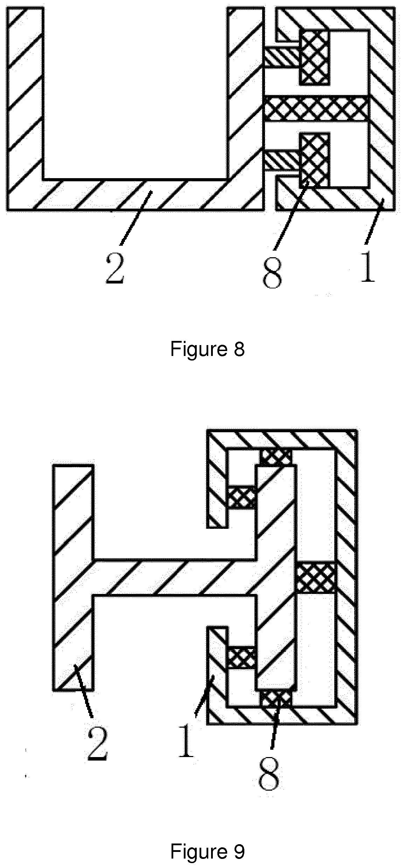

See Figure 8. In this embodiment, which is not forming part of the present invention, alternatively, the section of the second C-shaped arm is " 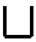 "; the section of the first C-shaped arm is " 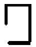 ", i.e. a rectangle with an opening on its side; there are two protruding parts on the side with an opening; there are three supporting devices between the side of the second C-shaped arm and the inner surface of the first C-shaped arm; two of the three supporting devices are respectively disposed on the side of the second C-shaped arm and stuck in the protruding parts; the third supporting device is disposed between the two supporting devices, so that the second C-shaped arm applies a pulling force to the first C-shaped arm through the two supporting devices and the second C-shaped arm applies pressure on the first C-shaped arm through the third supporting device.

See Figure 9. In this embodiment, which is not forming part of the present invention, alternatively, the section of the second C-shaped arm is " 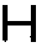 "; the section of the first C-shaped arm is " 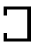 "; there are five supporting devices between the inner surfaces of the second C-shaped arm and the first C-shaped arm; the five supporting devices are respectively disposed on the upside, downside, upper left side, lower left side and right side of the part of the second C-shaped arm which is within the first C-shaped arm; the second C-shaped arm support the first C-shaped arm through the five supporting devices; besides, with the selection of materials of the supporting devices, such as ceramic, the supporting devices can produce the technical effect of friction reduction.

See Figure 5. In this embodiment, which has a structure of the C-shaped arms which is similar to the present invention, the first C-shaped arm 1 consists of two C-shaped beams; the second C-shaped arm 2; a chute which matches the C-shaped beam is disposed on the second C-shaped arm 2 to reduce the mass of the first C-shaped arm and enhance the accuracy of control over the first C-shaped arm, thus enhancing the precision of the biplanar X-ray imaging device.

In this embodiment, alternatively, the biplanar X-ray imaging device also comprises the driving device 9; the driving device 9 is disposed on the second C-shaped arm 2 to drive the first C-shaped arm 1 to slide along the second C-shaped arm; the first C-shaped arm 1 is driven by the driving device 9;
Alternatively, there are two driving devices 9 respectively disposed on the second C-shaped arm 2 to drive the first C-shaped arm 1 and the first image receiving device 5 respectively to slide along the second C-shaped arm 2. Preferably, the driving device 9 is a motor installed with gears on its rotor shaft; gear teeth which match the gears are disposed on the inner side of the first C-shaped arm 1, so that the first C-shaped arm is driven by the motor.

See Figure 1. In this embodiment, alternatively, the biplanar X-ray imaging device also comprises lifting and rotating components 10, an operating arm 11 and a mobile trolley 12; the sliding and rotating seat 3 is disposed on the operating arm 11 and can slide and rotate relative to the operating arm 11; the operating arm 11 is disposed on the mobile trolley 12 through the lifting and rotating components 10 to realize the multi-degree-of-freedom control over the first image receiving device 5 and the second image receiving device 6.

See Figure 2. In this embodiment, alternatively, the biplanar X-ray imaging device also comprises lifting and rotating components 10, an operating arm 11 and the first base 13; the sliding and rotating seat 3 is disposed on the operating arm 11 and can slide and rotate relative to the operating arm 11; the operating arm 11 is disposed on the first base 13 through the lifting and rotating components 10 to realize the multi-degree-of-freedom control over the first image receiving device 5 and the second image receiving device 6.

In this embodiment, alternatively, the biplanar X-ray imaging device also comprises lifting and rotating components 10, an operating arm 11 and the second base 14; the sliding and rotating seat 3 is disposed on the operating arm 11 and can slide and rotate relative to the operating arm 11; the operating arm 11 is disposed on the second base 14 through the lifting and rotating components 10; the second base 14 is slidably disposed on the ceiling to realize the multi-degree-of-freedom control over the first image receiving device 5 and the second image receiving device 6.

Finally, it should be noted that: The invention is defined in the appended claims and the above embodiments are used to illustrate the technical solutions of different biplanar X-ray imaging devices.

## Claims

1. A biplanar X-ray imaging device, comprising a first C-shaped arm (1), a second C-shaped arm (2), a sliding and rotating seat (3), a first X-ray generation device (4), a first image receiving device (5), a second X-ray generation device (6) and a second image receiving device (7);
the first C-shaped arm is slidably disposed outside the second C-shaped arm (2); the second C-shaped arm (2) is slidably disposed on the sliding and rotating seat (3);
the first X-ray generation device (4) is fixed on one end in the peripheral direction of the first C-shaped arm (1);
the first image receiving device (5) is disposed on the other end in the peripheral direction of the first C-shaped arm or slidably disposed on the inner side of the second C-shaped arm (2);
the second X-ray generation device and the second image receiving device are respectively fixed on the two ends in the peripheral direction of the second C-shaped arm,the intersecting line between the X-ray emission plane of the first X-ray generation device and the X-ray emission plane of the second X-ray generation device is a rotating axis;
the rotating axis is on a connecting line of the first X-ray generation device and the first image receiving device, in addition, the second X-ray generation device and the second image receiving device rotate along the rotating axis under the drive of the second C-shaped arm; **characterized in that** the first C-shaped arm (1) consists of two C-shaped beams and a chute is disposed on the second C-shaped arm (2), which matches the C-shaped beam of the first C-shaped arm.

2. A biplanar X-ray imaging device according to claim 1, **characterized in that** the biplanar X-ray imaging device also comprises a driving device (9); the driving device is disposed on the second C-shaped arm to drive the first C-shaped arm to slide along the second C-shaped arm;
alternatively, there are two driving devices (9) respectively disposed on the second C-shaped arm to drive the first C-shaped arm (1) and the first image receiving device (5) respectively to slide along the second C-shaped arm (2).

3. A biplanar X-ray imaging device according to claim 2, **characterized in that** the biplanar X-ray imaging device also comprises lifting and rotating components (10), an operating arm (11) and a mobile trolley (12); a sliding and rotating seat (3) is disposed on the operating arm and can slide and rotate relative to the operating arm (11); an operating arm is disposed on the mobile trolley through the lifting and rotating components (10).

4. A biplanar X-ray imaging device according to claim 2, **characterized in that** the biplanar X-ray imaging device also comprises lifting and rotating components, the operating arm (11) and a first base (13); a sliding and rotating seat is disposed on the operating arm and can slide and rotate relative to the operating arm; the operating arm is disposed on the first base through the lifting and rotating components.

5. A biplanar X-ray imaging device according to claim 2, **characterized in that** the biplanar X-ray imaging device also comprises lifting and rotating components, the operating arm (11) and a second base (14); a sliding and rotating seat is disposed on the operating arm and can slide and rotate relative to the operating arm; the operating arm is disposed on the second base through the lifting and rotating components; the second base is adapted to be slidably disposed on a ceiling.

## Patentansprüche

1. Biplan-Röntgenaufnahmevorrichtung, die einen ersten C-förmigen Arm (1), einen zweiten C-förmigen Arm (2), einen verschiebbaren und drehbaren Sitz (3), eine erste Röntgenstrahlen erzeugende Vorrichtung (4), eine erste Bildempfangsvorrichtung (5), eine zweite Röntgenstrahlen erzeugende Vorrichtung (6) und eine zweite Bildempfangsvorrichtung (7) umfasst;
der erste C-förmige Arm ist verschiebbar außerhalb des zweiten C-förmigen Arms (2) angeordnet;
der zweite C-förmige Arm (2) ist verschiebbar am verschiebbaren und drehbaren Sitz (3) angeordnet;
die erste Röntgenstrahlen erzeugende Vorrichtung (4) ist an einem Ende in der peripheren Richtung des ersten C-förmigen Arms (1) befestigt;
die erste Bildempfangsvorrichtung (5) ist am anderen Ende in der peripheren Richtung des ersten C-förmigen Arms oder verschiebbar auf der Innenseite des zweiten C-förmigen Arms (2) angeordnet;
die zweite Röntgenstrahlen erzeugende Vorrichtung und die zweite Bildempfangsvorrichtung sind jeweils an den beiden Enden in der peripheren Richtung des zweiten C-förmigen Arms befestigt, die Schnittlinie zwischen der Röntgenstrahlen abgebenden Ebene der ersten Röntgenstrahlen erzeugenden Vorrichtung und der Röntgenstrahlen abgebenden Ebene der zweiten Röntgenstrahlen erzeugenden Vorrichtung ist eine Drehachse;
die Drehachse liegt auf einer Verbindungslinie zwischen der ersten Röntgenstrahlen erzeugenden Vorrichtung und der ersten Bildempfangsvorrichtung, zusätzlich drehen sich die zweite Röntgenstrahlen erzeugende Vorrichtung und die zweite Bildempfangsvorrichtung entlang der Drehachse unter dem Antrieb des zweiten C-förmigen Arms; **dadurch gekennzeichnet, dass** der erste C-förmige Arm (1) aus zwei C-förmigen Balken besteht und ein Kanal am zweiten C-förmigen Arm (2) angebracht ist, der mit dem C-förmigen Balken des ersten C-förmigen Arms zusammenpasst.

2. Biplan-Röntgenaufnahmevorrichtung nach Anspruch 1, **dadurch gekennzeichnet, dass** die Biplan-Röntgenaufnahmevorrichtung auch eine Antriebsvorrichtung (9) umfasst; die Antriebsvorrichtung am zweiten C-förmigen Arm angebracht ist, um entlang dem zweiten C-förmigen Arm zu gleiten;
alternativ sind zwei Antriebsvorrichtungen (9) jeweils am zweiten C-förmigen Arm angebracht, um den ersten C-förmigen Arm (1) bzw. die erste Bildempfangsvorrichtung (5) jeweils entlang dem zweiten C-förmigen Arm (2) zu verschieben.

3. Biplan-Röntgenaufnahmevorrichtung nach Anspruch 2, **dadurch gekennzeichnet, dass** die Biplan-Röntgenaufnahmevorrichtung auch Hub- und Drehkomponenten (10), einen Betätigungsarm (11) und einen beweglichen Wagen (12) umfasst; ein verschiebbarer und drehbarer Sitz (3) am Betätigungsarm angeordnet ist und bezüglich des Betätigungsarms (11) verschiebbar und drehbar ist; ein Betätigungsarm am beweglichen Wagen über die Hub- und Drehkomponenten (10) angeordnet ist.

4. Biplan-Röntgenaufnahmevorrichtung nach Anspruch 2, **dadurch gekennzeichnet, dass** die Biplan-Röntgenaufnahmevorrichtung auch Hub- und Drehkomponenten, den Betätigungsarm (11) und einen ersten Sockel (13) umfasst; ein verschiebbarer und drehbarer Sitz am Betätigungsarm angeordnet und bezüglich des Betätigungsarms verschiebbar und drehbar ist; der Betätigungsarm am ersten Sockel über die Hub- und Drehkomponenten angeordnet ist.

5. Biplan-Röntgenaufnahmevorrichtung nach Anspruch 2, **dadurch gekennzeichnet, dass** die Biplan-Röntgenaufnahmevorrichtung auch Hub- und Drehkomponenten, den Betätigungsarm (11) und einen zweiten Sockel (14) umfasst; ein verschiebbarer und drehbarer Sitz am Betätigungsarm angeordnet und bezüglich des Betätigungsarms verschiebbar und drehbar ist; der Betätigungsarm am zweiten Sockel über die Hub- und Drehkomponenten angeordnet ist; der zweite Sockel zum verschiebbaren Anordnen an einer Decke ausgebildet ist.

## Revendications

1. Dispositif d'imagerie à rayons X à deux plans, comprenant un premier bras en forme de C (1), un second bras en forme de C (2), un siège coulissant et rotatif (3), un premier dispositif de génération de rayons X (4), un premier dispositif de réception d'image (5), un second dispositif de génération de rayons X (6) et un second dispositif de réception d'image (7) ;
le premier bras en forme de C est disposé de manière coulissante à l'extérieur du second bras en forme de C (2) ;
le second bras en forme de C (2) est disposé de manière coulissante sur le siège coulissant et rotatif (3) ;
le premier dispositif de génération de rayons X (4) est fixé sur une extrémité dans la direction périphérique du premier bras en forme de C (1) ;
le premier dispositif de réception d'image (5) est disposé sur l'autre extrémité dans la direction périphérique du premier bras en forme de C ou disposé de manière coulissante sur le côté interne du second bras en forme de C (2) ;
le second dispositif de génération de rayons X et le second dispositif de réception d'image sont respectivement fixés sur les deux extrémités dans la direction périphérique du second bras en forme de C, la ligne d'intersection entre le plan d'émission de rayons X du premier dispositif de génération de rayons X et le plan d'émission de rayons X du second dispositif de génération de rayons X est un axe de rotation ;
l'axe de rotation se trouve sur une ligne de liaison du premier dispositif de génération de rayons X et du premier dispositif de réception d'image, de plus le second dispositif de génération de rayons X et le second dispositif de réception d'image tournent le long de l'axe de rotation sous l'effet de l'entraînement du second bras en forme de C ;
**caractérisé par le fait que** le premier bras en forme de C (1) est constitué par deux poutres en forme de C et une glissière est disposée sur le second bras en forme de C (2), qui correspond à la poutre en forme de C du premier bras en forme de C.

2. Dispositif d'imagerie à rayons X à deux plans selon la revendication 1, **caractérisé par le fait que** le dispositif d'imagerie à rayons X à deux plans comprend également un dispositif d'entraînement (9) ; le dispositif d'entraînement est disposé sur le second bras en forme de C pour entraîner le premier bras en forme de C en coulissement le long du second bras en forme de C ;
en variante, il y a deux dispositifs d'entraînement (9) respectivement disposés sur le second bras en forme de C pour entraîner le premier bras en forme de C (1) et le premier dispositif de réception d'image (5) respectivement en coulissement le long du second bras en forme de C (2).

3. Dispositif d'imagerie à rayons X à deux plans selon la revendication 2, **caractérisé par le fait que** le dispositif d'imagerie à rayons X à deux plans comprend également des composants de levage et de rotation (10), un bras d'actionnement (11) et un chariot mobile (12) ; un siège coulissant et rotatif (3) est disposé sur le bras d'actionnement et peut coulisser et tourner par rapport au bras d'actionnement (11) ; un bras d'actionnement est disposé sur le chariot mobile par l'intermédiaire des composants de levage et de rotation (10).

4. Dispositif d'imagerie à rayons X à deux plans selon la revendication 2, **caractérisé par le fait que** le dispositif d'imagerie à rayons X à deux plans comprend également des composants de levage et de rotation, le bras d'actionnement (11) et une première base (13) ; un siège coulissant et rotatif est disposé sur le bras d'actionnement et peut coulisser et tourner par rapport au bras d'actionnement ; le bras d'actionnement est disposé sur la première base par l'intermédiaire des composants de levage et de rotation.

5. Dispositif d'imagerie à rayons X à deux plans selon la revendication 2, **caractérisé par le fait que** le dispositif d'imagerie à rayons X à deux plans comprend également des composants de levage et de rotation, le bras d'actionnement (11) et une seconde base (14) ; un siège coulissant et rotatif est disposé sur le bras d'actionnement et peut coulisser et tourner par rapport au bras d'actionnement ; le bras d'actionnement est disposé sur la seconde base par l'intermédiaire des composants de levage et de rotation ; la seconde base est adaptée pour être disposée de manière coulissante sur un plafond.
